# EUROPEAN PATENT APPLICATION

(11) **EP 4 450 629 A1**
(43) Date of publication of application: **23.10.2024**
(21) Application number: 22906135.3
(22) Date of filing: 10.11.2022
(51) Int. Cl.: C12N 15/74, C12N 15/09, C12N 1/21, C07K 14/235, A61K 39/10, C12P 21/00

(54) **WHOOPING COUGH ANTIGEN RECOMBINANT EXPRESSION VECTOR, AND ENGINEERED BACTERIUM THEREOF AND USE THEREOF**

(30) Priority: 17.12.2021 CN 202111550905
(71) Applicant: Grand Theravac Life Science (Nanjing) Co., Ltd., Nanjing, Jiangsu 210032 (CN)
(72) Inventor: GE, Jun, Nanjing, Jiangsu 210032 (CN); LI, Jianqiang, Nanjing, Jiangsu 210032 (CN); LI, Ling, Nanjing, Jiangsu 210032 (CN); ZHANG, Dayan, Nanjing, Jiangsu 210032 (CN); WANG, Xi, Nanjing, Jiangsu 210032 (CN); SHI, Yinghui, Nanjing, Jiangsu 210032 (CN); REN, Sulin, Nanjing, Jiangsu 210032 (CN); ZHOU, Tong, Nanjing, Jiangsu 210032 (CN)
(74) Representative: Uexküll & Stolberg
(86) International application number: PCT/CN2022/131069
(87) International publication number: WO 2023/109383

(57) **Abstract**

Provided are a recombinant expression vector for whooping cough antigens, an engineered bacterium and use thereof. The recombinant expression vector for whooping cough antigens comprises a protein expression cassette for pertactin, a gene for resistance screening, and an upstream recombinant nucleic acid fragment and a downstream recombinant nucleic acid fragment of filamentous haemagglutinin gene, wherein the protein expression cassette for pertactin is located between the upstream recombinant nucleic acid fragment and the downstream recombinant nucleic acid fragment of the filamentous haemagglutinin gene, and the upstream recombinant nucleic acid fragment and the downstream recombinant nucleic acid fragment of the filamentous haemagglutinin gene can undergo homologous recombination with the upstream and downstream of the filamentous haemagglutinin gene, respectively. The gene of FHA protein in the recombinant expression vector is knocked out and the copies PRN protein gene are increased, so that the *Bordetella pertussis* genetically engineered strain can efficiently overexpress PRN protein antigen and PT protein, and the expressed PRN protein antigen and PT protein exist in the cells and the supernatant respectively, thereby facilitating subsequent separation and purification of the antigens.

## Description

### Technical Field

The present invention belongs to the field of bio-pharmaceuticals, and relates to a recombinant expression vector for whooping cough antigens as well as an engineered strain and use thereof. Specifically, the present invention relates to a recombinant expression vector for over-expressing pertactin antigen and expressing pertussis toxin antigen, an engineered strain containing the same, and their uses in the preparation of pertactin and pertussis toxin antigens.

### Background Art

Whooping cough is a fulminating respiratory infectious disease caused by *Bordetella pertussis,* and extremely harmful to infants and young children. The clinical features are gradually worsening cough, typical paroxysmal and spasmodic cough, and deep, lasting and crowing-like inspiratory croup occurring at the end of cough. The course lasts for 2-3 months, so the disease is called whooping cough.

At present, vaccination is mainly adopted for prevention of whooping cough. The disease was well controlled with the first generation of whole cell pertussis vaccine (wPV). However, due to more severe adverse effects caused by the vaccination, wPV was gradually replaced with safer acellular pertussis vaccine (aPV). However, the incidences of whooping cough for adults and adolescents have risen since 2010. It is analyzed that, the major reasons are rapid decline of efficacy of aPV, and strain variation under selective pressure by vaccines, etc. Therefore, continuous improvement of whooping cough vaccines is an effective method for coping with the spread of whooping cough.

The *Bordetella pertussis* will produce multiple antigens, such as pertussis toxin (PT), filamentous haemagglutinin (FHA), pertactin (PRN) and lipopolysaccharide (PLS) during the growth process. The aPVs available in the domestic market are made by purifying a whooping cough antigen solution through a co-purification method, and adsorbing by an aluminum adjuvant following a treatment with a detoxication agent. Since the different antigenic components of *Bordetella pertussis* are not isolated separately, various antigens in whooping cough antigen solutions of different production batches have greater differences in terms of antigen proportion, thereby affecting the stability of vaccine quality.

Imported aPVs are the vaccines prepared by extracting the antigens, such as PT and FHA separately for separate detoxication via utilizing more technologically advanced column chromatography, and mixing these antigenic components according to a certain ratio. The process ensures batch-to-batch quality consistency and antigen purities of 90-95% of the vaccines, and is suitable for developing a DTacP-based combined vaccine (Hu Yeqin, et al., establishment of a novel purification process for acellular component pertussis vaccine). For example, the Chinese patent CN102793915B provides a three-component pertussis vaccine composition, namely, the purification was performed respectively to obtain PT, PRN and FHA, and then they were mixed according to a certain ratio. The literatures show that, FHA antigen is prone to degrade and brings interference to determination of purification effect. Multiple literatures (e.g., Wu Tengjie, Zhang Bin, Zhang Qing, et al., establishment of a novel purification process for acellular pertussis vaccine [J], Chinese Journal of Biologicals, 26 (01): 5-8) have reported a purification method for isolating FHA protein from PT protein. However, the steps for separately extracting PT protein are complicated.

The above methods have the problems of low expression levels of antigen proteins, and tedious separation and purification. Therefore, there is an urgent need for developing preparation methods for whooping cough antigens that can efficiently express the antigenic components and have a simplified separation and purification procedure.

### Disclosure of the Invention

To obtain PRN protein and PT protein as individual components more conveniently, a strain for producing whooping cough antigens is improved by the inventors through constructing a recombinant expression vector for whooping cough antigens, thereby providing a recombinant engineered strain in which FHA protein expression is blocked. The strain may simultaneously express PRN protein and PT protein, and has a shorter fermentation time. Further, the contents of the two proteins produced are significantly higher than those of the wild type strain, and the separation and purification are convenient.

In one aspect, the present invention provides a recombinant expression vector for whooping cough antigens, comprising a protein expression cassette for pertactin, a gene for resistance screening, and an upstream recombinant nucleic acid fragment and a downstream recombinant nucleic acid fragment of filamentous haemagglutinin gene, wherein the protein expression cassette for pertactin is located between the upstream recombinant nucleic acid fragment and the downstream recombinant nucleic acid fragment of filamentous haemagglutinin gene, and the upstream recombinant nucleic acid fragment and the downstream recombinant nucleic acid fragment of filamentous haemagglutinin gene can undergo homologous recombination with the upstream and downstream of filamentous haemagglutinin gene, respectively. The filamentous haemagglutinin gene is shown by SEQ ID NO: 1.

According to the present invention, the expression vector may be plasmid pUC57, pEASY-Blunt cloning vector, or a vector of pBBR series, preferably plasmid pUC57.

According to the present invention, the upstream recombinant nucleic acid fragment of filamentous haemagglutinin gene may comprise or be a nucleic acid fragment composed of continuous bases at position m-1000 in the nucleotide sequence shown by SEQ ID NO: 2, wherein m is a natural number smaller than or equal to 57. Preferably, the upstream recombinant nucleic acid fragment of filamentous haemagglutinin gene is a nucleic acid fragment composed of continuous bases at position 57-1000 in the nucleotide sequence shown by SEQ ID NO: 2.

According to an embodiment of the present invention, a nucleotide sequence of the protein expression cassette for pertactin is shown by SEQ ID NO: 3.

According to the present invention, the downstream recombinant nucleic acid fragment of filamentous haemagglutinin gene may comprise or be a nucleic acid fragment composed of continuous bases at position 1-n in the nucleotide sequence shown by SEQ ID NO: 4, wherein n is a natural number greater than or equal to 798-1000. Preferably, the downstream recombinant nucleic acid fragment of filamentous haemagglutinin gene is a nucleic acid fragment composed of continuous bases at position 1-798 in the nucleotide sequence shown by SEQ ID NO: 4.

According to the present invention, the gene for resistance screening may be one or more selected from a group consisting of a gene for kanamycin resistance screening, a gene for tetracycline resistance screening, a gene for ampicillin resistance screening, or a gene for chloromycetin resistance screening, preferably a gene for kanamycin resistance screening. Specifically, a nucleotide sequence of the gene for kanamycin resistance screening is shown by SEQ ID NO: 5 (Cloning vector pSN-caSAT1, genebank: MT001914.1); a nucleotide sequence of the gene for tetracycline resistance screening is shown by SEQ ID NO: 6 (Cloning vector pJC24, Genebank: KC442291.1); a nucleotide sequence of the gene for ampicillin resistance screening is shown by SEQ ID NO: 7 (Cloning vector pcDNA3-WSN-PB2, Genebank: MT966986.1); and a nucleotide sequence of the gene for chloromycetin resistance screening is shown by SEQ ID NO: 8 (Cloning vector pMYC, Genebank: MT572316.1).

In another aspect, the present invention further provides a recombinant expression engineered strain for whooping cough antigens, comprising the recombinant expression vector according to the present invention.

In still another aspect, the present invention further provides a preparation method of the recombinant expression engineered strain for whooping cough antigens, comprising transforming wild-type *Bordetella pertussis* competent cells by using the recombinant expression vector according to the present invention.

According to another embodiment of the present invention, the wild-type *Bordetella pertussis* is the wild-type *Bordetella pertussis* strain ATCC-BAA-589.

According to another embodiment of the present invention, the transformation is electrotransformation. Specifically, the electrotransformation may comprise linearizing the recombinant expression vector, and electro-transforming the wild-type *Bordetella pertussis* competent cells under conditions of 2500 V and 5 ms.

According to another embodiment of the present invention, the preparation method further comprises screening the transformed wild-type *Bordetella pertussis* competent cells by screening using the gene for resistance screening.

In yet another aspect, the present invention further provides a preparation method of whooping cough antigens, comprising fermentation by using the recombinant expression engineered strain for whooping cough antigens according to the present invention.

According to another embodiment of the present invention, the fermentation comprises the steps of:
(1) inoculating the recombinant expression engineered strain for whooping cough antigens according to the present invention into a plate containing Bordet-gengou blood agar medium, and culturing for 40-72 hours, preferably 48 hours;
(2) inoculating the cells obtained in step (1) into a shake flask containing MSS (Modified Stainer Scholte) medium, and culturing for 22.5-25 hours; and
(3) inoculating the bacterium solution obtained in step (2) into a fermentor containing MSS medium, and culturing for 28-30 hours.

Specifically, the whooping cough antigens are pertactin (PRN, its amino acid sequence shown by SEQ ID NO: 9) and/or pertussis toxin (PT, its amino acid sequence shown by SEQ ID NO: 10).
SEQ ID NO: 9: 
MNMSLSRIVKAAPLRRTTLAMALGALGAAPAAHADWNNQSIVKTGERQHGIHIQGSDPG
GVRTASGTTIKVSGRQAQGILLENPAAELQFRNGSVTSSGQLSDDGIRRFLGTVTVKAGKL
VADHATLANVGDTWDDDGIALYVAGEQAQASIADSTLQGAGGVQIERGANVTVQRSAI
VDGGLHIGALQSLQPEDLPPSRVVLRDTNVTAVPASGAPAAVSVLGASELTLDGGHITGG
RAAGVAAMQGAVVHLQRATIRRGDAPAGGAVPGGAVPGGAVPGGFGPGGFGPVLDGW
YGVDVSGSSVELAQSIVEAPELGAAIRVGRGARVTVSGGSLSAPHGNVIETGGARRFAPQ
AAPLSITLQAGAHAQGKALLYRVLPEPVKLTLTGGADAQGDIVATELPSIPGTSIGPLDVA
LASQARWTGATRAVDSLSIDNATWVMTDNSNVGALRLASDGSVDFQQPAEAGRFKVLT
VNTLAGSGLFRMNVFADLGLSDKLVVMQDASGQHRLWVRNSGSEPASANTLLLVQTPL
GSAATFTLANKDGKVDIGTYRYRLAANGNGQWSLVGAKAPPAPKPAPQPGPQPPQPPQP
QPEAPAPQPPAGRELSAAANAAVNTGGVGLASTLWYAESNALSKRLGELRLNPDAGGA
WGRGFAQRQQLDNRAGRRFDQKVAGFELGADHAVAVAGGRWHLGGLAGYTRGDRGF
TGDGGGHTDSVHVGGYATYIADSGFYLDATLRASRLENDFKVAGSDGYAVKGKYRTHG
VGASLEAGRRFTHADGWFLEPQAELAVFRAGGGAYRAANGLRVRDEGGSSVLGRLGLE
VGKRIELAGGRQVQPYIKASVLQEFDGAGTVHTNGIAHRTELRGTRAELGLGMAAALGR
GHSLYASYEYSKGPKLAMPWTFHAGYRYSW*
SEQ ID NO: 10:

MRCTRAIRQTARTGWLTWLAILAVTAPVTSPAWADDPPATVYRYDSRPPEDVFQNGFTA
WGNNDNVLDHLTGRSCQVGSSNSAFVSTSSSRRYTEVYLEHRMQEAVEAERAGRGTGH
FIGYIYEVRADNNFYGAASSYFEYVDTYGDNAGRILAGALATYQSEYLAHRRIPPENIRRV
TRVYHNGITGETTTTEYSNARYVSQQTRANPNPYTSRRSVASIVGTLVRMAPVIGACMAR
QAESSEAMAAWSERAGEAMVLVYYESIAYSF*MPIDRKTLCHLLSVLPLALLGSHVARAS
TPGIVIPPQEQITQHGGPYGRCANKTRALTVAELRGSGDLQEYLRHVTRGWSIFALYDGT
YLGGEYGGVIKDGTPGGAFDLKTTFCIMTTRNTGQPATDHYYSNVTATRLLSSTNSRLCA
VFVRSGQPVIGACTSPYDGKYWSMYSRLRKMLYLIYVAGISVRVHVSKEEQYYDYEDAT
FETYALTGISICNPGSSLC*MLRRFPTRTTAPGQGGARRSRVRALAWLLASGAMTHLSPAL
ADVPYVLVKTNMVVTSVAMKPYEVTPTRMLVCGIAAKLGAAASSPDAHVPFCFGKDLK

RPGSSPMEVMLRAVFMQQRPLRMFLGPKQLTFEGKPALELIRMVECSGKQDCP*MKSPR
RACWSAASPPNWAPRPAARTRTCRSASARISSVPAAVPWKSCCAPSSCNNGRCACFWVP
SNSLSKASPRSN*MQRQAGLPLKANPMHTIASILLSVLGIYSPADVAGLPTHLYKNFTVQE
LALKLKGKNQEFCLTAFMSGRSLVRACLSDAGHEHDTWFDTMLGFAISAYALKSRIALT
VEDSPYPGTPGDLLELQICPLNGYCE*MLINNKKLLHHILPILVLALLGMRTAQAVAPGIVI
PPKALFTQQGGAYGRCPNGTRALTVAELRGNAELQTYLRQITPGWSIYGLYDGTYLGQA
YGGIIKDAPPGAGFIYRETFCITTIYKTGQPAADHYYSKVTATRLLASTNSRLCAVFVRDG
QSVIGACASPYEGRYRDMYDALRRLLYMIYMSGLAVRVHVSKEEQYYDYEDATFQTYA
LTGISLCNPAASIC*

According to the present invention, by constructing and using the recombinant expression vector for whooping cough antigens, a *Bordetella pertussis* genetically engineered strain is obtained, wherein the gene of FHA protein is knocked out, and the copy of PRN protein gene is increased, so that the *Bordetella pertussis* genetically engineered strain can efficiently over-express PRN protein antigen and express PT protein simultaneously. In addition, the PRN protein antigen and PT protein expressed by the *Bordetella pertussis* genetically engineered strain provided by the present invention exist in the cells and the supernatant of fermentation liquor respectively, thereby further facilitating subsequent antigen isolation and purification.

Compared with some existing reports, the *Bordetella pertussis* engineered strain OEPRN-PT provided by the present invention may simultaneously obtain PRN protein and PT protein with higher expression levels, and shows significant advantages. For example, an expression strain with two copies of PRN gene promoted by an FHA promoter was prepared in the O'Connor Lab, with a final PRN protein concentration of about 186.7 µg/ml (Loosmore S M, Yacoob R K, Zealey G R, et al., Hybrid genes over-express pertactin from Bordetella pertussis [J]. Vaccine, 1995, 13(6): 571), while the PRN expression concentration produced by the engineered strain OEPRN-PT provided by the present invention is 418-555 µg/ml.

Through the examples below, the present invention has verified the beneficial effects as follows:
1. By knocking out the FHA gene to block the expression of FHA, the effect of FHA protein on PT protein is eliminated in the purification process of antigens from pertussis strain; and as compared with a wild strain, the PT protein content is increased, while the purified PT protein has less protein impurities.
2. With an increased copy number of PRN, the yield of PRN is significantly increased; and as compared with the PRN content in the strain having two copies in the existing report, the PRN protein content of the present invention is significantly increased.
3. Compared with the wild type, the engineered strain OEPRN-PT can complete the growth by fermentation within about 28-30 hours, and the time is shortened by over 25%.
4. The PRN protein expressed by the engineered strain OEPRN-PT of the present invention is concentrated in the cells, and the PT protein is concentrated in the supernatant of the bacterium solution. However, both the two proteins produced by the wild strain are concentrated in the supernatant of the bacterium solution. Thus, the proteins produced by the engineered strain OEPRN-PT of the present invention are more suitable for subsequent separation and purification.

In conclusion, in the present invention, through one pertussis engineered strain, high contents of PRN protein and PT protein can be produced simultaneously, and the proteins are easy to be isolated and purified, thereby meeting the requirements for industrial production of antigens.

### Brief Description of the Drawings

The embodiments of the present invention will be described in detail below in combination with the drawings.
Fig. 1 is a schematic diagram of plasmid construction of the recombinant vector p U C5 7 - Fhup- Prn - Fhdown - Kan;
Fig. 2 shows the SDS-PAGE electrophoretic analysis results of digestion identification after the resistance gene Kan is ligated to the downstream of the fragment Fhup-Prn-Fhdown in the vector, and the resistance gene Kan is about 963 bp, wherein M: Marker; Lane 1# and Lane 2#: parallel samples carrying the resistance gene Kan;
Fig. 3 shows the identification of the upstream and downstream insertions in the recombinant expression vector through verification at a DNA level, wherein M: Marker; Lanes 1-6: confirmation of upstream insertion site; Lane 1'-6': confirmation of downstream insertion site;
Fig. 4 is an electrophoretogram for identification of FHA gene knockout through verification at a DNA level, wherein M: Marker; Lanes 1-6: FHA gene detection;
Fig. 5 shows the result of PRN protein produced by fermentation of the engineered strain OEPRN-PT, which is purified through a QHP chromatographic column with Buffer B as an eluent;
Fig. 6 shows the detection results of PRN protein purified through a QHP chromatographic column by using HPLC;
Fig. 7 shows the results of PT protein produced by fermentation of the engineered strain OEPRN-PT, which is purified through an SP-inspire chromatographic column with Buffer D as an eluent; and
Fig. 8 shows the results of PT protein produced by fermentation of the wild strain WT, which is purified through an SP-inspire chromatographic column with Buffer D as an eluent.

### Best modes for carrying out the Invention

The present invention will be further described below in combination with the specific examples. The advantages and characteristics of the present invention will be clearer in view of the description.

Unless otherwise specified, the experimental methods described in the present invention are all conventional methods. Unless otherwise specified, all biological materials are commercially available.

### Example 1 Construction of recombinant vector pUC57-Fhup-Prn-Kan-Fhdown

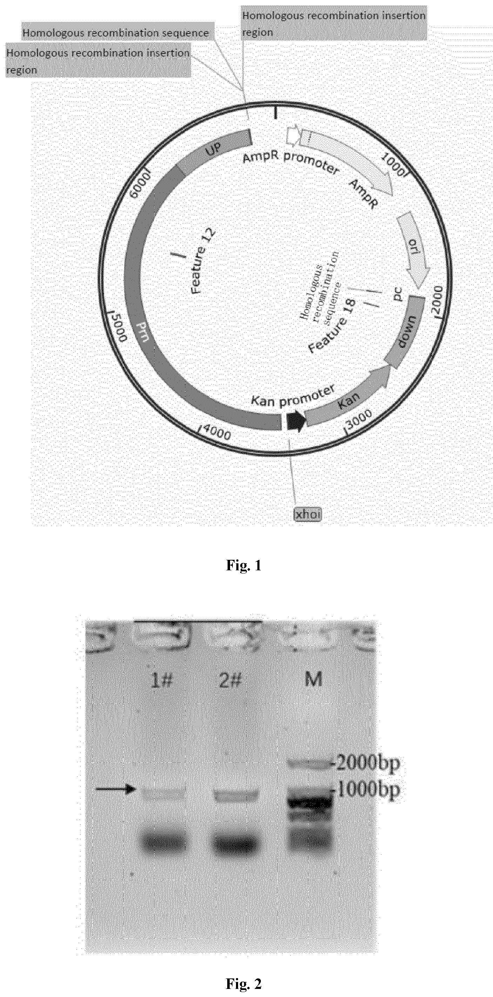

The recombinant vector pUC57-Fhup-Prn-Kan-Fhdown was constructed according to the schematic diagram of plasmid construction shown in Fig. 1.

### 1.1 Construction of the fragment Fhup-Prn-Kan-Fhdown for the vector

The fragments of various sequence needed for construction of the fragment Fhup-Prn-Kan-Fhdown for the vector and the primers thereof are shown in Table 1. The fragments were amplified respectively and then assembled through an overlap PCR, which can be used for obtaining two copies of PRN gene.

### 1.2 Construction of the recombinant vector pUC57-Fhup-Prn-Kan-Fhdown

The vector pUC57 was digested with the restriction enzymes Hind III and Bam HI, and the digestion system had a volume of 200µl (purchased from NEB) and is comprised 10 µl of 10 ×2.1 buffer, 15 µg of the plasmid, 4 µl of Hind III, 4 µl of Bam HI, and H₂O (q.s. to 200µl). The digestion was conducted at 37°C for 2 h. Then, the detection was conducted by using 1% agarose electrophoresis, and a target band was recovered. The digested pUC57 was ligated into the Fhup-Prn-Kan-Fhdown prepared in step 1.1 with a recombinase (purchased from Vazyme). The constructed recombinant vector pUC57-Fhup-Prn-Kan-Fhdown was transformed into *Escherichia coli* DH5a competent cells (purchased from Beijing TransGen Biotech) and then subjected to a colony PCR detection with the primers pt-Kan-F and pt-Kan-CZR, respectively (shown in Table 2). The results (shown in Fig. 2) show that, the Kan resistance fragment was ligated accurately and about 963 bp, indicating that the recombinant expression vector pUC57-Fhup-Prn-Kan-Fhdown was constructed successfully. Finally, the positive clones which were identified correct through the colony PCR were sent to Shanghai Sangon for sequencing, and the clone having a nucleotide sequence which is completely consistent with that of the target gene was preserved at -80°C.

**Table 1 Various fragments and primers thereof needed for construction of Fhup-Prn-Kan-Fhdown**

| Fragment to be constructed | Fragment as needed | Sequence of fragment | Sequence of primer |
|---|---|---|---|
| Fhup-Prn-Kan-Fhdown | Fh-up | SEQ ID NO: 2 | tgagagtgcaccaTATGAAGCTTGGGAATGCCGAT GTCG (SEQ ID NO: 11) |
| | | | ATTCCGACCAGCGAAGTG (SEQ ID NO: 12) |
| | Prn | SEQ ID NO: 3 | CACTTCGCTGGTCGGAATATGAACATGTCT CTGTCAC (SEQ ID NO: 13) |
| | | | CGTCCCGTTCTCGGTCAAGGCATTTACCAG CTGTACCGGTAG (SEQ ID NO: 14) |
| | Fh-down | SEQ ID NO: 4 | cgagttcttctgaCATTCCACCATCGAGAG (SEQ ID NO: 15) |
| | | | gctcacatgTGGATCCCCTCGGCAGCCTTGACC A (SEQ ID NO: 16) |
| | Kan | SEQ ID NO: 5 | ACCGAGAACGGGACGGTCACCATATCGGC CGATTCGGCCGTGCTCGAGccggaattgccagctg (SEQ ID NO: 17) |
| | | | GTGGAATGtcagaagaactcgtcaagaag (SEQ ID NO: 18) |

**Table 2 Primers for Colony PCR**

| Primer name | Sequence of primer |
|---|---|
| pt-kan-F | GTGGAATGtcagaagaactcgtcaagaag (SEQ ID NO: 19) |
| pt-kan-CZR | GGCCGATTCGGCCGTGCTCGAGccggaattgccagctg (SEQ ID NO: 20) |

### Example 2 Preparation of Bordetella pertussis genetically engineered strain

The original wild-type *Bordetella pertussis* strain (hereinafter referred to as the wild strain WT) used in the present example is *Bordetella pertussis* wild-type strain ATCC-BAA-589, which was purchased from BeNa Culture Collection.

2.1 Culture of cells: the wild-type strain ATCC-BAA-589 (glycerol stock) ready to be competent was activated. A bacterium solution comprising an appropriate amount of the wild-type strain ATCC-BAA-589 was coated onto a plate containing Bordet-gengou medium, and cultured in an incubator at 37°C for 48 h and then taken out. The bacterial plaques on the surface were scraped off by a sampling rod, and then inoculated into 100 ml MSS medium and cultured for 24 h. Afterwards, the value OD₆₀₀ was measured and controlled between 1 and 3.

2.2 Preparation of competent cells: the cells prepared in step 2.1 were collected, centrifuged at 4°C for 15 min, and then resuspended with 100 ml of pre-cooled distilled water at 4°C. The cells were washed twice and then centrifuged at 4°C for 15 min. The cells were collected, and resuspended and washed with a proper volume of 10% sterile glycerin. The cells were centrifuged at 4°C for 15 min and then collected, and resuspended with 1 ml of 10% sterile glycerin to obtain competent cells of the wild-type strain ATCC-BAA-589. The competent cells were preserved in a refrigerator at -70°C.

2.3 Preparation of transformation fragment: the recombinant expression vector pUC57-Fhup-Pm-Fhdown-Kan prepared in Example 1 was linearized with restriction enzyme Hind III at the upstream of the fragment Fhup.

2.4 Electrotransformation and homologous recombination: 2µg of the linearized fragment DNA was transferred into the competent cells of the wild-type strain ATCC-BAA-589 prepared in step 2.2 under conditions of 2500 V and 5 ms. After a liquid culture for 24 h, the competent cells were coated onto a solid medium containing kanamycin (can be replaced with tetracycline, ampicillin or chloromycetin according to the resistance gene correspondingly) and cultured for 3-5 days. Single clones were picked for verification.

### Example 3 Identification of Bordetella pertussis genetically engineered strain

### 3.1 Verification at a DNA level

### 3.1.1 Experimental procedure

The single clones prepared in Example 2 were picked into a 1.5 ml EP tube containing 10 µl of MSS, from which 2 µl was sucked up as a template to perform a PCR verification. Meanwhile, the single clones were subjected to streak culture.

**Table 3 Primers used for DNA verification**

| Primer name | Sequence | Size |
|---|---|---|
| Primers for verifying upstream insertion site | GATGTCCTTGTTGGACATGC (SEQ ID NO: 21) | 3729 |
| | attccggCTCGAGCACGGCCGAATCG (SEQ ID NO: 22) | |
| Primers for verifying downstream insertion site | TCGTACGAGTACTCCAAGGGCCCGAAG (SEQ ID NO: 23) | 1964 |
| | GCGAATCTTGTTGGAGAAACGGGTGT (SEQ ID NO: 24) | |
| Primers for verifying FHA gene knockout | CCTGATGTTGGCGTGTACGGGTCTT (SEQ ID NO: 25) | 484 |
| | GATCGTGACGGTGCCCTGTTGGA (SEQ ID NO: 26) | |

### 3.1.2 Experimental results

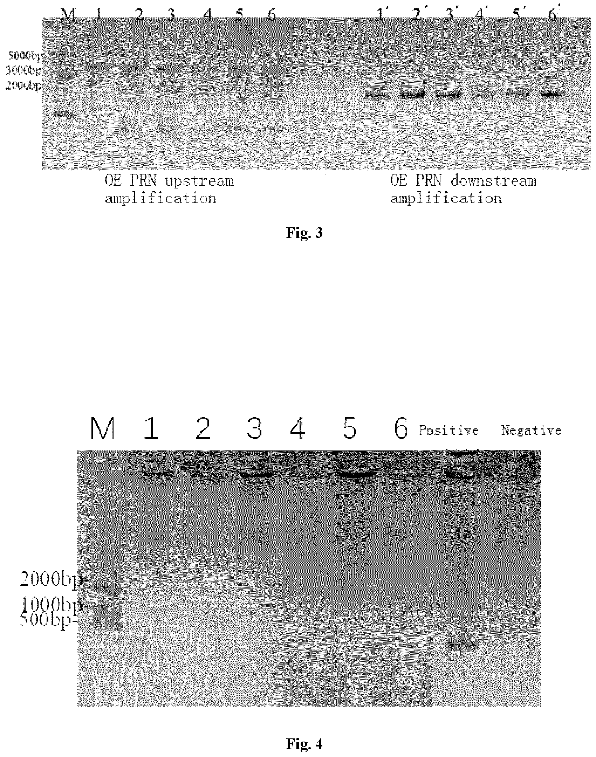

The verification results show that, the upstream insertion site and the downstream insertion site are all correct (Fig. 3), and the FHA gene was identified as being knocked out (Fig. 4), that is, the engineered strain OEPRN-PT was constructed successfully (in the engineered strain OEPRN-PT of the present invention, the PRN gene was added through recombination, the FHA gene was partially knocked out, and FHA protein was not expressed).

### 3.2 Verification of growth and fermentation situations

### 3.2.1 Experimental procedure

100 µL of the engineered strain OEPRN-PT verified to be correct in step 3.1 was inoculated into two plates containing Bordet-Gengou blood agar medium (operated in parallel) and cultured at 37°C for about 48 h. A microscopic examination showed no bacterial contamination. Bacterial lawn was scraped off from the plates and inoculated into 300 ml of MSS medium, and the value OD₆₀₀ was measured as 2.12. A microscopic examination showed no bacterial contamination. After the culture, 300 ml of the bacterium solution was taken and inoculated into a fermentor filled with 3 L of MSS medium. The initial fermentation conditions included 35°C, 150 rpm and aeration rate of 2 L/min, and the dissolved oxygen was controlled at 40% during the fermentation process. When the fermentation was performed for 29 h, the culture was terminated according to the rebound of dissolved oxygen, and the cell precipitate was collected.

### 3.2.2 Experimental results

The engineered strain OEPRN-PT and the wild strain WT were fermented respectively, and their results were compared and analyzed (shown in Table 4). The growth time of the wild strain WT was about 36-42 hours, and the growth time of the engineered strain OEPRN-PT in the fermentor was about 28-30 hours which was shortened by over 25%. The PRN protein produced by the engineered strain OEPRN-PT had a concentration of 418-555 µg/ml, while the PRN protein was not detected when the cells of the wild strain were collected. The PT protein produced by the engineered strain OEPRN-PT had a concentration of about 6.23-8.98 µg/ml, which was significantly increased as compared with the concentration of the PT protein of the wild strain, i.e., 3.5 µg/ml.

**Table 4 Comparison of culture parameters and culture results at various stages of fermentation processes for Bordetella pertussis**

| Strain | Growth time in primary plate (h) | Growth time in secondary shake flask (h) | OD₆₀₀ value when inoculation into secondary shake flask | OD₆₀₀ value when inoculation into fermentor | Growth time in fermentor (h) | OD₆₀₀ value at the end of fermentation | PRN concentration when the cells were collected (µg/ml) | PT concentration when the cells were collected (µg/ml) |
|---|---|---|---|---|---|---|---|---|
| Wild strain WT | 48 | 25 | 1.77-2.24 | 0.12-0.2 | 36-42 | 5.45-7.76 | / | 3.5 |
| Engineered strain OEPRN-PT | 48 | 22.5-25 | 1.96-3.08 | 0.17-0.266 | 28-30 | 7.18-8.15 | 418-555 | 6.23-8.98 |

The above results show that, the engineered strain OEPRN-PT obtained in the present invention greatly shortens the fermentation time, and the fermentation liquor has higher expression levels of the target proteins at the end of fermentation, wherein PRN protein has an extremely high expression level, and the expressed PT protein has a higher concentration compared with that of the wild strain WT.

### Example 4 Purification of PRN protein obtained by engineered strain OEPRN-PT

### 4.1 Experimental materials

Buffer for cell disruption: 10 mM of Tris-HCl, 150 mM of NaCl, and 1 mM of PMSF (phenylmethylsulfonyl fluoride)
Buffer for redissolving: 35 mM of NaCl, and 25 mM of Tris-HCl
Buffer A: 50 mM of Tris-HCl
Buffer B: 50 mM of Tris-HCl, and 1 M of NaCl

### 4.2 Experimental procedure

### 4.2.1 Crude extraction and purification of PRN protein

The fermentation liquor of the engineered strain OEPRN-PT obtained in Example 3 was centrifuged at 4°C for 30 min to obtain a cell precipitate. Then the cell precipitate was redissolved in 1000 ml of buffer for cell disruption, incubated at 60°C for 1 h followed by being centrifuged at 4°C for 30 min. The supernatant was collected, and 950 ml of extract liquid was collected totally. An aqueous solution of ammonium sulfate was added into the extract liquid for precipitating for 2 h. After standing at a room temperature for 1 h, the solution was centrifuged at 4°C for 50 min, and the precipitate was collected.

The precipitate obtained through salting-out by ammonium sulfate was redissolved in 600 ml of buffer for redissolving. After redissolving overnight, the solution was centrifuged at 4°C for 50 min, and 500 ml of the supernatant was collected totally. The supernatant was subjected to ultrafiltration by Pellicon XL PXB10C50 ultrafiltration membrane package and exchanged into 50 mM of Tris-HCl to obtain 260 ml of ultrafiltrate.

### 4.2.2 Refinement of PRN protein

A QHP chromatographic column was washed with 0.5 M NaOH aqueous solution and Buffer B in sequence for regeneration, and then equilibrated with 5 column volumes of Buffer A at a flow rate of 2ml/min. The ultrafiltrate obtained in step 4.2.1 was loaded onto the QHP column at a flow rate of 2ml/min. After flowing through was complete, the column was washed with 5 column volumes of Buffer A.

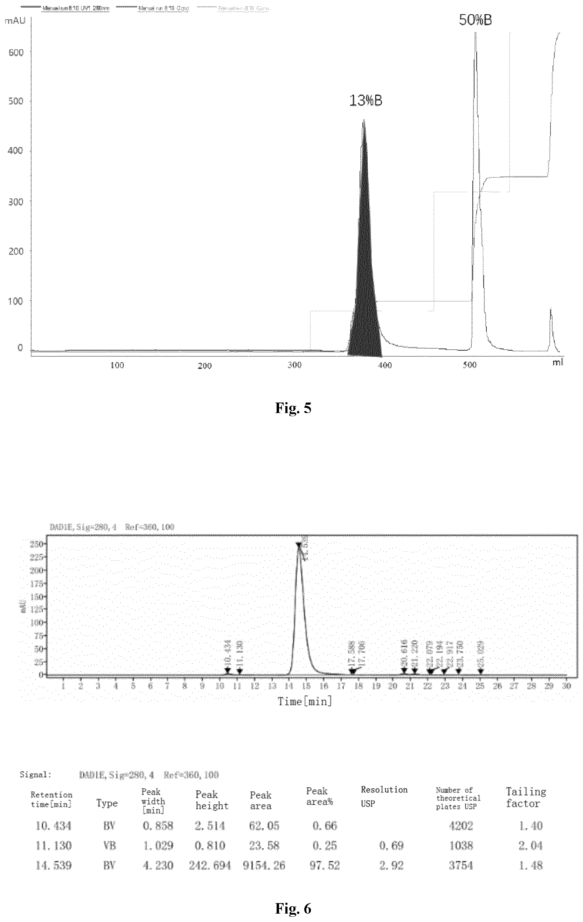

Then, the column was eluted with 13% (volume ratio) of Buffer B, and an elution peak was collected. PRN protein was in the elution peak and named as QHP-13%B (Fig. 5). There was hardly any impure protein in the elution sample. The collected protein was detected by a BCA method and HPLC, respectively.

### 4.3 Experimental results

The BCA detection results are shown in Table 5. The concentration of PRN protein varies over the collection time and the highest concentration may reach 655.5223 (µg/ml). The concentration at each stage is basically higher than 194 µg/ml. The HPLC results show that (as shown in Fig. 6), the concentration of PRN protein may reach 97.52%.

**Table 5 Concentration of purified PRN determined by BCA**

| Protein name | Protein concentration (µg/ml) |
|---|---|
| Tube 1 | 194.5504 |
| Tube 2 | 655.5223 |
| Tube 3 | 249.784 |
| Tube 4 | 194.1351 |

### Example 5 Purification of PT protein obtained by engineered strain OEPRN-PT

### 5.1 Experimental procedure

### Experimental materials:

Buffer C: 50 mM PB (phosphate buffer solution) + 2M urea
Buffer D: 50 mM PB + 1M NaCl + 2M urea

### 5.1.1 Crude extraction and purification of PT protein

The fermentation liquor of the engineered strain OEPRN-PT obtained in Example 3 was centrifuged at 4°C for 30 min, and the supernatant was remained. After the filtered supernatant was concentrated with a 10kDa membrane package, the impurities were removed by a filtering membrane. Then, ultrafiltration was conducted so that the solution was changed into 50 mM PB, and 300 ml of ultrafiltrate was obtained totally.

### 5.1.2 Refinement of PT protein

An SP-inspire chromatographic column was washed with 0.5 M aqueous solution of NaOH and Buffer D in sequence for regeneration, and then balanced to a baseline with Buffer C with a flow rate of 2ml/min. 145 ml of ultrafiltrate was loaded into the SP-inspire chromatographic column with a flow rate of 2ml/min. After flowing through was completed, the chromatographic column was washed subsequently to the baseline with Buffer C, and the impurities were washed out with 8% (volume ratio) of Buffer D.

The protein was eluted with 20% (volume ratio) Buffer D, and an elution peak was collected. PT protein was in the elution peak and named as SP-20%B.

### Purification of PT protein obtained by the wild strain WT:

The fermentation liquor obtained after the fermentation of the wild strain WT was taken, and the purification procedure was the same as the operations in Sections 5.1.1 and 5.1.2.

### 5.2 Experimental results

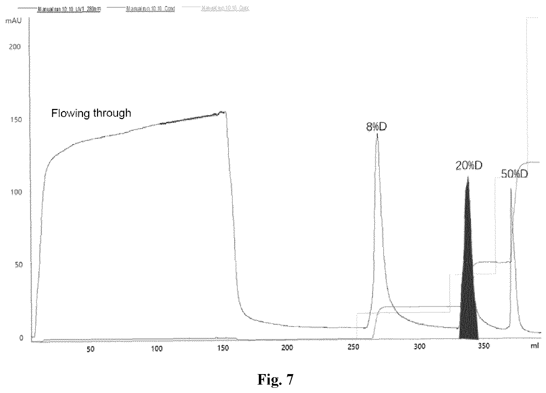

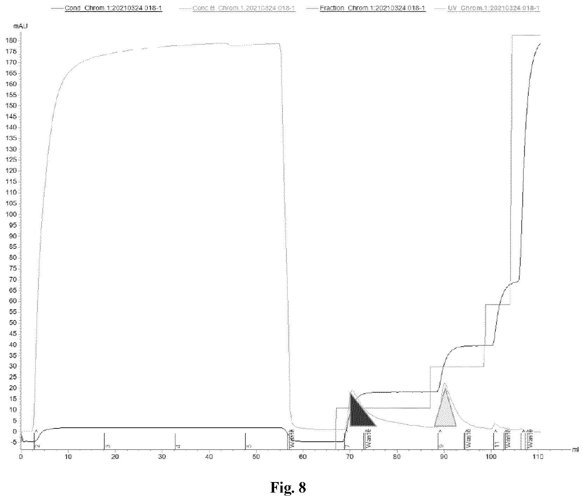

The purification results of PT protein produced by the engineered strain OEPRN-PT and PT protein produced by the wild strain WT are shown as Figs. 7 and 8, respectively. It can be obviously seen from Figs. 7 and 8 that, when PT protein was obtained by purifying the engineered strain OEPRN-PT through an ion chromatography, there is no FHA protein contamination in the elution sample and the PT content is higher. However, when PT is obtained by purifying the strain WT through the ion chromatography, there is FHA protein contamination in the elution sample and the PT content is lower.

To sum up, in the present invention, by constructing a recombinant expression vector for blocking FHA protein expression and introducing the same into a wild-type pertussis strain, the pertussis engineered strain OEPRN-PT which over-expresses PRN protein is provided. This strain can produce high expression levels of PRN protein and PT protein, simultaneously. The strain has a short fermentation time, the contents of PRN protein and PT protein of which are obviously higher than those of the wild strain. Moreover, the two proteins are produced in the cell precipitate and the supernatant of bacterium solution respectively, and convenient to be isolated and purified, thereby meeting the requirements for industrial production.

Although the present invention has been described in detail above, those skilled in the art should understand that, various modifications and changes may be made to the present invention without departing from the spirit and scope of the present invention. The scope of the present invention is not limited to the detailed descriptions above, but should be subject to the claims.

The above description is merely the preferred examples of the present invention, rather than a limitation to the present invention in any way. Although the present invention has been disclosed through the preferred examples above, the present invention is not limited by the examples. A few changes or modifications may be made by those skilled who are familiar with this filed by utilizing the technical contents disclosed above without departing from the scope of the technical solutions of the present invention, so as to form equivalent examples having equivalent changes. However, for the contents which do not depart from the technical solutions of the present invention, any modification or equivalent changes and modifications made to the above examples according to the technical substance of the present invention should still fall within the scope of the technical solutions of the present invention.

## Claims

1. A recombinant expression vector for whooping cough antigens, comprising a protein expression cassette for pertactin, a gene for resistance screening, an upstream recombinant nucleic acid fragment and a downstream recombinant nucleic acid fragment of filamentous haemagglutinin gene, wherein the protein expression cassette for pertactin is located between the upstream recombinant nucleic acid fragment and the downstream recombinant nucleic acid fragment of filamentous haemagglutinin gene, and the upstream recombinant nucleic acid fragment and the downstream recombinant nucleic acid fragment of filamentous haemagglutinin gene can undergo homologous recombination with the upstream and downstream of filamentous haemagglutinin gene, respectively.

2. The recombinant expression vector according to claim 1, wherein the expression vector is plasmid pUC57, pEASY-Blunt cloning vector, or a vector of pBBR series, preferably plasmid pUC57.

3. The recombinant expression vector according to claim 1 or 2, wherein the upstream recombinant nucleic acid fragment of filamentous haemagglutinin gene comprises or is a nucleic acid fragment composed of continuous bases at positions m-1000 in the nucleotide sequence shown by SEQ ID NO: 2, wherein m is a natural number smaller than or equal to 57; preferably, the upstream recombinant nucleic acid fragment of filamentous haemagglutinin gene is a nucleic acid fragment composed of continuous bases at positions 57-1000 in the nucleotide sequence shown by SEQ ID NO: 2;
preferably, the downstream recombinant nucleic acid fragment of filamentous haemagglutinin gene comprises or is a nucleic acid fragment composed of continuous bases at positions 1-n in the nucleotide sequence shown by SEQ ID NO: 4, wherein n is a natural number greater than or equal to 798-1000; more preferably, the downstream recombinant nucleic acid fragment of filamentous haemagglutinin gene is a nucleic acid fragment composed of continuous bases at positions 1-798 in the nucleotide sequence shown by SEQ ID NO: 4.

4. The recombinant expression vector according to any one of claims 1-3, wherein the nucleotide sequence of the protein expression cassette for pertactin is shown by SEQ ID NO: 3.

5. The recombinant expression vector according to any one of claims 1-4, wherein the gene for resistance screening is one or more selected from a group consisting of a gene for kanamycin resistance screening, a gene for tetracycline resistance screening, a gene for ampicillin resistance screening, or a gene for chloromycetin resistance screening, preferably a gene for kanamycin resistance screening;
preferably, a nucleotide sequence of the gene for kanamycin resistance screening is shown by SEQ ID NO: 5; a nucleotide sequence of the gene for tetracycline resistance screening is shown by SEQ ID NO: 6; a nucleotide sequence of the gene for ampicillin resistance screening is shown by SEQ ID NO: 7; and a nucleotide sequence of the gene for chloromycetin resistance screening is shown by SEQ ID NO: 8.

6. A recombinant expression engineered strain for whooping cough antigens, comprising the recombinant expression vector according to any one of claims 1-5.

7. A preparation method of the recombinant expression engineered strain for whooping cough antigens according to claim 6, comprising transforming wild-type *Bordetella pertussis* competent cells by using the recombinant expression vector according to any one of claims 1-5;
preferably, the wild-type *Bordetella pertussis* is the wild-type *Bordetella pertussis* strain ATCC-BAA-589;
preferably, the transformation is electrotransformation; more preferably, the electrotransformation comprises linearizing the recombinant expression vector according to any one of claims 1-5 and electro-transforming the wild-type *Bordetellapertussis* competent cells under conditions of 2500 V and 5 ms.

8. The preparation method according to claim 7, further comprising screening the transformed wild-type *Bordetellapertussis* competent cells by screening using the gene for resistance screening.

9. A preparation method of whooping cough antigens, comprising fermentation by using the recombinant expression engineered strain for whooping cough antigens according to claim 6.

10. The preparation method according to claim 9, wherein the fermentation comprises the following steps of:
(1) inoculating the recombinant expression engineered strain for whooping cough antigens according to claim 6 into a plate containing Bordet-gengou blood agar medium, and culturing for 40-72 hours, preferably 48 hours;
(2) inoculating the cells obtained in step (1) into a shake flask containing MSS medium, and culturing for 22.5-25 hours; and
(3) inoculating the bacterium solution obtained in step (2) into a fermentor containing MSS medium, and culturing for 28-30 hours.

11. The preparation method according to claim 9 or 10, wherein the whooping cough antigens are pertactin and/or pertussis toxin.
